(19) **Europäisches Patentamt**
**European Patent Office**
**Office européen des brevets**

(11) **EP 3 750 620 B1**

(12) **EUROPÄISCHE PATENTSCHRIFT**

(45) Veröffentlichungstag und Bekanntmachung des Hinweises auf die Patenterteilung:
**30.03.2022 Patentblatt 2022/13**

(21) Anmeldenummer: **20178660.5**

(22) Anmeldetag: **08.06.2020**

(51) Internationale Patentklassifikation (IPC):
**B01D 61/02** (2006.01)  **B01D 71/06** (2006.01)
**C07C 67/38** (2006.01)  **C07C 67/56** (2006.01)
**C07C 69/24** (2006.01)  **C07C 67/03** (2006.01)

(52) Gemeinsame Patentklassifikation (CPC):
(C-Sets verfügbar)
**B01D 61/027; C07C 67/03; C07C 67/38; C07C 67/56**  (Forts.)

(54) **VERFAHREN ZUR HERSTELLUNG EINES ESTERS DURCH ALKOXYCARBONYLIERUNG**

METHOD OF PRODUCING AN ESTER BY MEANS OF ALCOXYCARBONYLATION

PROCÉDÉ DE FABRICATION D'UN ESTER PAR ALCOXYCARBONYLATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.06.2019 EP 19179570**

(43) Veröffentlichungstag der Anmeldung:
**16.12.2020 Patentblatt 2020/51**

(73) Patentinhaber: **Evonik Operations GmbH**
**45128 Essen (DE)**

(72) Erfinder:
- **Kucmierczyk, Peter**
  **44652 Herne (DE)**
- **Franke, Robert**
  **45772 Marl (DE)**
- **Fridag, Dirk**
  **45721 Haltern am See (DE)**
- **Knossalla, Johannes**
  **46514 Schermbeck (DE)**
- **Schäpertöns, Marc**
  **45657 Recklinghausen (DE)**
- **Gluth, Frederik**
  **45472 Mülheim an der Ruhr (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**WO-A1-2013/107902    WO-A1-2015/110843**

- **PATRIZIA MARCHETTI ET AL: "Molecular Separation with Organic Solvent Nanofiltration: A Critical Review", CHEMICAL REVIEWS, Bd. 114, Nr. 21, 21. Oktober 2014 (2014-10-21), Seiten 10735-10806, XP055618006, US ISSN: 0009-2665, DOI: 10.1021/cr500006j**

(52) Gemeinsame Patentklassifikation (CPC): (Forts.)

C-Sets
**C07C 67/03, C07C 69/24;**
**C07C 67/38, C07C 69/24;**
**C07C 67/56, C07C 69/24**

C-Sets

**Beschreibung**

Die vorliegende Erfindung betrifft ein Verfahren zur Herstellung eines Esters durch Alkoxycarbonylierung eines C2- bis C16-Kohlenwasserstoffs mit mindestens einer Mehrfachbindung, vorzugsweise mit mindestens einer olefinischen Doppelbindung, bei dem das eingesetzte homogene Katalysatorsystem mittels Membrantrennung aus dem Produktgemisch abgetrennt und zur Reaktionszone zurückgeführt wird. In einer Weiterbildung der vorliegenden Erfindung wird der so gebildete Ester durch Umesterung zu einem anderen Ester umgesetzt.

Die Alkoxycarbonylierung ist die Umsetzung eines Kohlenwasserstoffs, der mindestens eine Mehrfachbindung, vorzugsweise mindestens eine olefinische Doppelbindung aufweist, mit Kohlenmonoxid und einem Alkohol zu den entsprechenden Estern. Dabei werden üblicherweise Metall-Ligand-Komplexe als Katalysator eingesetzt, die homogen gelöst im Reaktionsgemisch und damit auch im Produktgemisch vorliegen. Um den gewünschten Ester aus dem Produktgemisch isolieren zu können, ist es von Vorteil zuvor zumindest den Großteil an homogen gelösten Katalysator aus dem Produktgemisch zu entfernen. Auf der anderen Seite sind die Kosten für die eingesetzten Katalysatoren vergleichsweise hoch, weshalb der Katalysator auch schon aus wirtschaftlichen Gründen zurückgewonnen werden sollte.

Für die Abtrennung des homogen gelösten Katalysators vom restlichen Reaktionsgemisch sind verschiedene Verfahren bekannt. Die WO 2013/107902 A1 offenbart beispielsweise ein Verfahren, bei dem der homogen gelöste Katalysator nach Alkoxycarbonylierung mittels einer Membran abgetrennt wird, die für Moleküle ab einem Molekülgewicht von 1 kDa undurchlässig ist, wodurch der Katalysator zurückgehalten und im Retentat angereichert wird. Gemäß der WO 2013/107902 A1 sind auf Polyimiden oder Polydimethylsiloxan basierende Membranmaterialien bevorzugt.

Die in der WO 2013/107902 A1 offenbarten Materialien weisen aber den Nachteil auf, dass sie oft keine ausreichende Langzeitstabilität vor allem gegenüber Säuren aufweisen, welche als Co-Katalysatoren typischerweise Teil des Katalysatorsystems sind, und bei mehrfacher Beanspruchung instabil werden können. Weiterhin weisen diese Membranmaterialien keinen nennenswerten Rückhalt für den eingesetzten Kohlenwasserstoff oder das Lösungsmittel auf, welches gleichzeitig Edukt bei der Alkoxycarbonylierung sein kann, beispielsweise der eingesetzte Alkohol. Weder Alkohol noch Kohlenwasserstoff werden im Retentat angereichert. Dadurch müssen nachgeschaltete Aufarbeitungs- und/oder Aufreinigungsschritte, wie nachfolgende Destillationsschritte, aufwendiger betrieben werden, um das Lösungsmittel bzw. die Edukte vom Produkt abzutrennen.

Die daraus resultierende Aufgabe der vorliegenden Erfindung war die Bereitstellung eines Verfahrens zur Alkoxycarbonylierung, bei der der homogene Katalysator unter Verwendung eines säurestabilen Membranmaterials, welches auch über einen längeren Zeitraum eingesetzt werden kann, abgetrennt wird. Weiterhin war die Aufgabe ein Alkoxycarbonylierungsverfahren bereitzustellen, wobei bei der Membranabtrennung bevorzugt die gebildeten Produkte durchgelassen und zumindest für einen Teil der eingesetzten Edukte, insbesondere den Alkohol, zumindest teilweise zurückgehalten werden.

Die zugrundeliegende Aufgabe der vorliegenden Erfindung konnte durch Verfahren nach Anspruch 1 gelöst werden. Bevorzugte Ausführungsformen sind in den Unteransprüchen angegeben.

Erfindungsgemäß umfasst das Verfahren zur Herstellung eines Esters durch Carbonylierung eines C2- bis C16-Kohlenwasserstoffs, wobei das Verfahren die folgenden Schritte umfasst:

a) Umsetzen (Carbonylieren) eines C2- bis C16-Kohlenwasserstoffs mit mindestens einer olefinischen Doppelbindung mit Kohlenmonoxid und mit einem Alkohol, welcher ein Mono- oder Polyalkohol (zwei oder mehr OH-Gruppen) mit 1 bis 50 Kohlenstoffatomen oder eine Mischung aus zwei oder mehr Mono- und/oder Polyalkoholen ist und welcher, wenn es sich um einen Monoalkohol handelt, in einem Molverhältnis zum C2- bis C16-Kohlenwasserstoff (Alkohol: C2- bis C16-Kohlenwasserstoff) von 2 bis 20 eingesetzt wird oder welcher, wenn es sich um einen Polyalkohol handelt, in einem Molverhältnis zum eingesetzten Kohlenwasserstoff (Kohlenwasserstoff: Polyalkohol) von 2 bis 20 eingesetzt wird, in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer Reaktionszone unter Erhalt eines flüssigen Produktgemisches, welches zumindest den durch die Umsetzung gebildeten Ester, das homogene Katalysatorsystem, Leichtsieder, und/oder Hochsieder und nicht umgesetzte Alkohole umfasst;

b) Durchführen einer Membrantrennung zur Abtrennung des homogenen Katalysatorsystems aus dem flüssigen Produktgemisch, wodurch das homogene Katalysatorsystem und zusätzlich nicht umgesetzter Kohlenwasserstoff und/oder nicht umgesetzter Alkohol, vorzugsweise nicht umgesetzter Alkohol im Retentat angereichert werden und der in Schritt a) gebildete Ester im Permeat angereichert wird, wobei als Membranmaterial ein säurestabiles, d. h. ein mindestens 300 h in Anwesenheit der Säure des Katalysatorsystems stabiles OSN-fähiges (Organic Solvent Nanofiltration) Membranmaterial, welches zumindest eine trennaktive Schicht aufweist, die aus PEEK (Polyetheretherketon) besteht, eingesetzt wird, und Rückführung des Retentats in die Reaktionszone;

c) Aufarbeitung des Permeat mittels eines oder mehrerer Trennschritte, ausgewählt aus thermischer Trennung, Extraktion, Kristallisation oder Membrantrennung, vorzugsweise mittels eines oder mehrerer Destillationsschritte, zur Abtrennung des in Schritt a) gebildeten Esters und zur Abtrennung des nicht umgesetzten C2- bis C16-Kohlenwasserstoffs mit mindestens einer olefinischen Doppelbindung und/oder des nicht umgesetzten Alkohols und Rückführung des nicht umgesetzten C2- bis C16-Kohlenwasserstoffs mit mindestens einer olefinischen Doppelbindung und/oder des nicht umgesetzten Alkohols in die Reaktionszone von Schritt a.

Die bei der Umsetzung in Schritt a) eingesetzten Kohlenwasserstoffe müssen mindestens eine Mehrfachbindung aufweisen. Bevorzugt sind Kohlenwasserstoffe mit mindestens einer olefinischen Doppelbindung, besonders bevorzugt sind Kohlenwasserstoffe mit einer olefinischen Doppelbindung. Grundsätzlich ist die Anzahl der Kohlenstoffatome von Verbindung, die mindestens eine Mehrfachbindung, vorzugsweise mindestens eine olefinische Doppelbindung aufweisen, nicht begrenzt. Technische relevant sind aber nur die Carbonylierung von C2- bis C16-Kohlenwasserstoffen mit mindestens einer Mehrfachbindung, vorzugsweise mindestens einer olefinischen Doppelbindung. In einer bevorzugten Ausführungsform der vorliegenden Erfindung können C3- bis C12-Kohlenwasserstoffe, vorzugsweise mit mindestens einer olefinischen Doppelbindung eingesetzt werden. Darunter fallen insbesondere n-Alkene, iso-Alkene, Cycloalkene und aromatische Alkene mit 2 bis 16 Kohlenstoffatomen, vorzugsweise 3 bis 12 Kohlenstoffatomen.

Die vorgenannt beschriebenen Kohlenwasserstoffe können zusätzlich zu der mindestens einen olefinischen Doppelbindungen eine oder mehrere weitere funktionelle Gruppen enthalten. Beispiele für geeigente funktionelle Gruppen sind Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl-, Amino-, Ether-, Thioether-, Aryl-, Heteroaryl- oder Silylgruppen und/oder Halogensubstituenten.

Besonders bevorzugte Kohlenwasserstoffe, die in Schritt a) des erfindungsgemäßen Verfahrens eingesetzt werden, weisen nur eine olefinische Doppelbindung auf, insbesondere n-Alkene und iso-Alkene mit 2 bis 16 Kohlenstoffatomen, vorzugsweise 3 bis 12 Kohlenstoffatomen. Die eingesetzten Kohlenwasserstoffe sind vorzugsweise unsubstituiert.

Die eingesetzten und vorher beschriebenen Kohlenwasserstoffe mit olefinischer Doppelbindung werden erfindungsgemäß mit Kohlenmonoxid (CO) und einem Alkohol zum entsprechenden Ester in Schritt a) umgesetzt. Das Kohlenmonoxid kann dabei direkt als Einsatzgemisch oder durch die Zugabe eines Kohlenmonoxid-haltigen Gases, ausgewählt aus Synthesegas, Wassergas, Generatorgas und anderen Kohlenmonoxid-haltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei der Umsetzung in Schritt a) eingesetzte Alkohol ist ein Mono- oder Polyalkohol (zwei oder mehr OH-Gruppe) mit 1 bis 50 Kohlenstoffatomen, vorzugsweise 1 bis 15 Kohlenstoffatomen, besonders bevorzugt 1 bis 10 Kohlenstoffatomen oder eine Mischung aus zwei oder mehr Mono- und/oder Polyalkoholen. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol oder Mischungen daraus, vorzugsweise Ethanol, 1-Propanol, 1-Butanol, 1-Pentanol, 1-Hexanol, 2-Propanol, tert-Butanol, 3-Pentanol, 2-Propylheptanol.

Der in Schritt a) eingesetzte Alkohol wird, wenn es sich um einen Monoalkohol handelt, in einem Molverhältnis zum eingesetzten Kohlenwasserstoff (Monoalkohol : Kohlenwasserstoff) von 2 bis 20, vorzugsweise von 3 bis 10 und besonders bevorzugt von 4 bis 6 eingesetzt. Der Monoalkohol wird somit - bezogen auf den eingesetzten Kohlenwasserstoff - im molaren Überschuss hinzugefügt. Dadurch kann der Alkohol sowohl als Edukt für die Carbonylierung als auch als Lösemittel fungieren. Der in Schritt a) eingesetzte Alkohol wird, wenn es sich um einen Polyalkohol handelt, in einem Molverhältnis zum eingesetzten Kohlenwasserstoff (Kohlenwasserstoff : Polyalkohol) von 2 bis 20, vorzugsweise von 3 bis 10 und besonders bevorzugt von 4 bis 8 eingesetzt. Der Polyalkohol wird bezogen auf den eingesetzten Kohlenwasserstoff also im molaren Unterschuss hinzugefügt.

Die erfindungsgemäße Umsetzung in Schritt a) wird in Gegenwart eines homogenen Katalysatorsystems durchgeführt, welches zumindest ein Metall aus der Gruppe 8 bis 10 des Periodensystems der Elemente (PSE) oder eine Verbindung davon, einen phosphorhaltigen Liganden und eine Säure als Co-Katalysator umfasst.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [$PdCl_2$], Palladium(II)-Acetylacetonat [$Pd(acac)_2$], Palladium(II)-Acetat [$Pd(OAc)_2$], Dichloro-(1,5-cyclooctadien)palladium(II) [$Pd(cod)_2Cl_2$], Bis(dibenzylideneaceton)palladium(0) [$Pd(dba)_2$], Tris(dibenzylideneaceton)dipalladium(0) [Pd2(dba)$_3$] Bis(acetonitril)-dichloropalladium(II) [$Pd(CH_3CN)zCl_2$], Palladium(cinnamyl)-dichlorid [Pd(cinna-

myl)Cl$_2$]. Vorzugsweise kommen die Verbindungen [Pd(acac)$_2$] oder [Pd(OAc)$_2$] zum Einsatz. Die Metallkonzentration von Palladium in Schritt a) beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für die das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann für die beschriebene Umsetzung in Schritt a) 1 : 1 bis 10 : 1, vorzugsweise 2 : 1 bis 6 : 1, besonders bevorzugt 3 : 1 bis 5 : 1 betragen.

Das homogene Katalysatorsystem umfasst weiterhin eine Säure, wobei es sich insbesondere um eine Brönsted- oder eine Lewis-Säure handelt. Als Lewis-Säure können insbesondere Lewis Säuren mit einem LAU-Wert von mehr als 25, vorzugsweise mit einem LAU-Wert von 29. Der LAU-Wert ist eine neue Methode zur Bestimmung der Stärke von Lewis-Säuren (JR Gaffen et al., Chem, Vol. 5, Issue 6, S. 1567-1583). Als Lewis-Säure werden vorzugsweise Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs $\leq$ 5, besonders bevorzugt eine Säurestärke von pKs $\leq$ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure.

Die Umsetzung bzw. Carbonylierung des eingesetzten Kohlenwasserstoffs mit olefinischer Doppelbindung in Schritt a) wird vorzugsweise bei einer Temperatur von 25 bis 140 °C, weiterhin bevorzugt bei einer Temperatur von 80 bis 130 °C und besonders bevorzugt bei einer Temperatur von 90 bis 120 °C durchgeführt. Der Druck in Schritt a) kann zwischen 5 und 60 bar, vorzugsweise zwischen 10 und 40 bar, besonders bevorzugt zwischen 15 und 30 bar betragen.

Die beschriebene Umsetzung in Schritt a) findet in einer geeigneten Reaktionszone statt. Die Reaktionszone für die Umsetzung umfasst mindestens einen Reaktor, kann aber auch aus zwei oder mehr Reaktoren bestehen. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein.

Durch die oben beschriebene Umsetzung in Schritt a) wird ein flüssiges Produktgemisch erhalten, die zumindest den durch die Umsetzung gebildeten Ester, das homogene Katalysatorsystem, nicht umgesetzte Alkohole A, und ggf. weitere Komponenten wie Leichtsieder, beispielsweise leichtsiedende Nebenprodukte wie Ether, und/oder Hochsieder und/oder nicht umgesetzte Kohlenwasserstoffe umfasst. Das Produktgemisch wird dann der nachfolgenden Membrantrennung in Schritt b) zugeführt. Bei der Umsetzung in Schritt a) kann zudem ein Abgas, welches zumindest aus unreaktiven Verunreinigungen wie Stickstoff, Wasserstoff, Alkanen und leichtsiedenden Nebenprodukten (beispielsweise die bereits genannten Ether) aus der Reaktionszone entnommen werden. Die Verunreinigungen und leichtsiedenden Nebenprodukte könnten sich akkumulieren und den Partialdruck des Reaktionsgases (CO) auf lange Sicht erniedrigen, wodurch die Reaktion verlangsamt werden könnte.

Im nachfolgenden Schritt b) wird das Produktgemisch einer Membrantrennung zugeführt, um das homogene Katalysatorsystem vom Produktgemisch abzutrennen. Durch das erfindungsgemäße Membranmaterial werden das homogene Katalysatorsystem und nicht umgesetzter Kohlenwasserstoff und/oder nicht umgesetzter Alkohol im Retentat angereichert, während der in Schritt a) gebildete Ester im Permeat angereichert wird. Das Permeat, welches den gebildeten Ester enthält, wird dann zum nachfolgenden Schritt c) geführt. Das Retentat, welches das angereicherte homogene Katalysatorsystem umfasst, wird dann in die Reaktionszone zurückgeführt. Bei der Rückführung des Retentats kann weiterhin ein Purgestrom, welcher inerte Alkane, leichtsiedende Nebenprodukte (bspw. Ether), mögliche Abbauprodukte des Katalysatorsystems oder andere Verunreinigungen enthalten kann, die durch die eingesetzten Kohlenwasserstoffströme eingetragen werden, wie beispielsweise Spuren von Wasser oder Stickstoff, entnommen werden, um eine Akkumulation in der oder den Reaktionszonen zu vermeiden. Die Rückführung des Retentats sorgt dafür, dass das bei der Membrantrennung im Retentat anfallende Katalysatorsystem zur Reaktion zurückgeführt wird. Dadurch werden Katalysatorverluste durch Abscheidung oder Desaktivierung minimiert und das Verfahren kostengünstiger gestaltet. Katalysatorverluste sind meistens nicht ganz zu vermeiden, aber die erwähnte Verringerung der Verluste führt dazu, dass weniger Katalysator durch Zuführung von frischem Katalysator ersetzt werden muss.

Die Membrantrennung beruht auf der Semipermeabilität des Membranmaterials, welches für bestimmte Stoffe durchlässig und für andere Stoffe undurchlässig ist. Das in Schritt b) des erfindungsgemäßen Verfahrens eingesetzte Membranmaterial ist ein OSN-Membranmaterial (OSN = Organic Solvent Nanofiltration). Ein solches Membranmaterial besteht vorzugsweise zumindest aus einer eher dünnen trennaktiven Schicht (auch: aktiven Trennschicht) und optional einem dickeren Backing, worauf sich die trennaktive Schicht befindet. Bevorzugt besteht das erfindungsgemäße Membranmaterial zumindest aus einer trennaktiven Schicht und einem Backing. Zwischen trennaktiver Schicht und Backing können eine oder mehrere Zwischenschichten vorhanden sein. In einer bevorzugten Ausführungsform besteht das Membranmaterial nur aus der trennaktiven Schicht und dem Backing. Das Membranmaterial, aus zumindest trennaktiver Schicht und Backing, sollte säurestabil sein, damit das Membranmaterial nicht durch die im flüssigen Produktgemisch als Co-Katalysator befindliche Säure beschädigt wird. Der Begriff "säurestabil" meint im Rahmen der vorliegenden Erfindung, dass das Membranmaterial mindestens 300 h in Anwesenheit der Säure des Katalysatorsystems, insbesondere einer Brönsted-Säure mit einem $pKs \leq 5$, besonders bevorzugt mit einem $pKs \leq 3$ oder einer Lewis-Säure mit einem LAU-Wert von mehr als 25, vorzugsweise mit einem LAU-Wert von 29, stabil ist und nicht zerstört wird, wodurch die eigentliche Trennwirkung nicht mehr erzielt werden könnte.

Das Backing weist insbesondere eine poröse Struktur auf, die für das durch die trennaktive Schicht gelangte Permeat durchlässig ist. Das Backing hat eine stabilisierende Funktion und dient als Träger für die trennaktiven Schicht. Das Backing kann grundsätzlich aus jedem geeigneten porösen Material bestehen. Voraussetzung ist jedoch, dass das Material säure- und basenstabil ist. Das Backing kann auch aus dem gleichen Material bestehen wie die trennaktive Schicht.

Die erfindungsgemäß trennaktive Schicht besteht aus PEEK (Polyetheretherketon).

Als trennaktive Schicht können insbesondere PEEK-Polymere mit einem Sulfonierungsgrad von weniger als 20%, besonders bevorzugt mit einem Sulfonierungsgrad von weniger als 10% eingesetzt werden. Die entsprechenden PEEK-Polymere und deren Herstellung sind in der WO 2015/110843 A1 oder in J. da Silva Burgal et al.; Journal of Membrane Science, Vol. 479 (2015), S. 105-116 beschrieben. Es hat sich überraschend gezeigt, dass dieses Material besonders stabil ist, insbesondere auch gegenüber der Säure als Co-Katalysator des homogenen Katalysatorsystems. Außerdem zeichnet sich das erfindungsgemäße PEEK-Material dadurch aus, dass es als trennaktive Schicht eingesetzt die gebildeten Ester durchlässt, während selbst die eingesetzten Eduktalkohole zumindest teilweise zurückgehalten werden und sich dadurch im Retentat anreichern. Dadurch kann die nachfolgende Aufarbeitung des restlichen flüssigen Produktgemisches wirtschaftlicher und nachhaltiger betrieben werden, weil verglichen mit bekannten Membranmaterialien weniger Alkohole abgetrennt werden müssen.

Die Membrantrennung in Schritt b) wird vorzugsweise Temperatur von 25 bis 100°C, weiterhin bevorzugt 30 bis 80°C und besonders bevorzugt 40 bis 70°C durchgeführt. Um das Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wodurch ein anderer Strom innerhalb des erfindungsgemäßen Verfahrens erhitzt wird. Optional ist auch ein Degasingschritt zwischen der Reaktionszone in Schritt a) und der Membrantrennung in Schritt b) vorhanden, um vorher leicht flüchtige Verbindungen wie Kohlenmonoxid und/oder restliche, über das Abgas nicht abgetrennte unreaktiven Verunreinigungen wie Stickstoff, Wasserstoff, Alkane und leichtsiedenden Nebenprodukte (beispielsweise die bereits genannten Ether) aus der Produktmischung zu entfernen. Dabei wird das Produktgemisch erst unter den Partialdruck der gelösten Komponenten wie Kohlenmonoxid entspannt, so dass diese ausgasen, um dann wieder den Druck zu erhöhen, der in der Membrantrennung vorgesehen ist.

Der Transmembrandruck (TMP) kann in Schritt b) 10 bis 60 bar, vorzugsweise 15 bis 55 bar, besonders bevorzugt 20 bis 50 bar betragen (relativ). Der permeatseitige Druck kann dabei oberhalb des atmosphärischen Drucks bis 15 bar, vorzugsweise 3 bis 7 bar betragen, woraus sich anhand des TMP dann der retentaseitige Druck ergibt. In einer bevorzugten Ausführungsform sollte bei den Druckverhältnissen und insbesondere beim permeatseitigen Druck darauf geachtet werden, dass der Druck in Abhängigkeit von dem eingesetzten Kohlenwasserstoff, dem eingesetzten Alkohol und der vorhandenen Temperatur eingestellt wird, um eine Verdampfung nach dem Durchtritt durch die Membran zu vermeiden, da dadurch die gesamte Fahrweise instabil werden könnte. Gleiches gilt grundsätzlich auch für gelöste Komponenten wie Kohlenmonoxid, die optional durch den bereits genannten Degasingschritt entfernt werden können.

Die Ökonomie von Membrantrennverfahren kann sich stark nach der Lebensdauer der verwendeten Membranmaterialien bemessen, weshalb auch die Lebensdauer bzw. die Stabilität der Membran ein Kriterium für die Auswahl des geeigneten Membranmaterials sein kann. Dabei wird eine Mindestlebensdauer von etwa einem halben Jahr angenommen. Das kann insbesondere für Prozesse, wie dem vorliegenden Verfahren, bei denen das Produkt im Permeat angereichert wird, relevant werden, weil die erforderliche Membranfläche mit der Gesamtkapazität des Verfahrens steigt.

Daraus ergeben sich insbesondere drei Kriterien für die Lebensdauer bzw. die Stabilität der Membran, nämlich die technische Integrität der Membran, der Membranrückhalt (entspricht der Trennleistung der Membran, siehe nachfolgende Definition) und die Permeabilität der Membran (siehe nachfolgende Definition). Fehler im Hinblick auf die technische Integrität, wie die Auflösung der Membran, die Ausbildung von Fehlstellen oder Löchern oder eine deutliche Schrumpfung oder Vergrößerung der Membranfläche, können dabei auf den Rückhalt der Membran (Rückhalt sinkt) und die Permeabilität (Permeabilität steigt an) ausstrahlen und auch dafür sorgen, dass die Membran nicht mehr mit dem genannten Druck beaufschlagt werden kann und/oder sich der Transmembrandruck nicht einstellen kann.

In der Membrantechnik wird zur Charakterisierung der Durchlässigkeit bzw. der Trennleistung einer Membran der Rückhalt R der Membran hinsichtlich einer bestimmten Komponente des Stoffgemisches gemäß nachfolgender Formel (1) definiert:

$$R = 1 - w_{(I)P} / w_{(I)R} \qquad (1),$$

worin $w_{(I)P}$ für den Massenanteil der betrachteten Komponente im Permeat und $w_{(I)R}$ für den Massenanteil der betrachteten Komponente im Retentat der Membran steht. Der Rückhalt kann somit Werte von 0 bis 1 annehmen und wird deswegen gerne in % angegeben. Ein Rückhalt von 0 % bedeutet, dass die betrachtete Komponente ungehindert durch die Membran permeiert, sodass die Massenanteile der Komponenten im Retentat wie im Permeat gleich sind. Andererseits bedeutet ein Rückhalt von 100 %, dass die betrachtete Komponente vollständig von der Membran zurückgehalten wird, was sich technisch jedoch kaum realisieren lässt.

Neben dem Rückhalt ist auch die sogenannte Permeabilität der Membran für die Charakterisierung ihrer Durchlässigkeit gemäß der nachfolgenden Formel (2) maßgeblich:

$$P = m' / (A * TMP) \qquad (2),$$

worin m' für den Massenstrom des Permeats, A für die Fläche der Membran und TMP für den angelegten Transmembrandruck steht. Angegeben wird die Permeabilität in der Regel in der Einheit $kg /(h * m^2 * bar)$. Die Permeabilität ist somit eine Kennzahl, die auf die Membranfläche und den eingestellten TMP normiert ist.

Im Hinblick auf die Charakterisierung der Stabilität einer Membran kann eine relative Änderung der Permeabilität $P_{Rel}$ gemäß der nachfolgenden Formel (3) definiert werden:

$$P_{Rel} = P_{t=x} / P_{t=0} \qquad (3),$$

worin $P_{t=x}$ für die Permeabilität zum Zeitpunkt t=x und $P_{t=0}$ für die ursprüngliche Permeabilität zum Zeitpunkt t=0 steht (möglich ist auch ein anderer zeitlicher Bezugspunkt, mit der Maßgabe, dass t=x > t=y).

Eine Membran kann weiterhin über die Selektivität bezüglich einer bestimmten Komponente charakterisiert werden. Die Selektivität bezeichnet das Verhältnis der Konzentrationen einer bestimmen Komponente i vor und nach der Membran. Die Selektivität berechnet sich nach der Formel (4):

$$S_i = C_{P\_i} / C_{F\_i} \qquad (4),$$

worin $C_{F\_i}$ für die Konzentration der betrachteten Komponente i im Feed zur Membran, hier das flüssige Produktgemisch, und $C_{P\_i}$ für die Konzentration der betrachteten Komponente i im Permeat steht.

Nach einer bevorzugten Ausführungsform der vorliegenden Erfindung weist das erfindungsgemäße Membranmaterial nach einem Einsatz im hier beschriebenen Membrantrennverfahren in Schritt b) von 300 Stunden die folgenden beiden Merkmale auf:

• der Membranrückhalt R für das homogene Katalysatorsystem beträgt mindestens 75%, vorzugsweise 85%, besonders bevorzugt 90% und verschlechtert sich um nicht mehr als 30 Prozentpunkte, vorzugsweise nicht mehr als 20 Prozentpunkte, besonders bevorzugt nicht mehr als 10 Prozentpunkte; oder

• die relative Permeabilität $P_{rel}$ weist einen Wert zwischen 30% und 300%, vorzugsweise zwischen 50% und 150%, besonders vorzugsweise zwischen 75% und 125% auf.

Die Membran sollte folglich nicht ihre technische Integrität verlieren. Beispiele für den Verlust der technischen Integrität sind die Auflösung der Membran, die Ausbildung von Fehlstellen/Löchern und/oder eine erhebliche Schrumpfung oder eine erhebliche Vergrößerung der Membranfläche. Das erfindungsgemäße Membranmaterial soll dabei insbesondere gegenüber der Anwesenheit von Säuren in einer Menge von 0,1 Gew.-% bis 5 Gew.-% stabil sein, nicht ihre technische Integrität verlieren und die oben genannten beiden Merkmale aufweisen.

Das Permeat aus der Membrantrennung (Schritt b)) wird im nachfolgenden Schritt c) einem Trennverfahren ausgewählt aus der Gruppe, bestehend aus einer thermischen Trennung, beispielsweise Destillation, einer Extraktion, einer Kristallisation oder einer weiteren Membrantrennung, unterworfen, um den in Schritt a) gebildeten Ester vom restlichen Permeat abzutrennen. Das Trennverfahren ist vorzugsweise eine Destillation. Die entsprechenden Verfahrensbedingungen sind dem Fachmann bekannt.

Es kann vorkommen, dass bei dem in Schritt c) verwendeten Trennverfahren, insbesondere bei der Destillation, nicht nur der gebildete Ester aus dem Permeat abgetrennt wird, sondern auch die möglicherweise entstandenen Hochsieder, beispielsweise hochsiedende Nebenprodukte, die bei der Umsetzung in Schritt a) anfallen können. Um diese Hochsieder zu entfernen kann das erfindungsgemäße Verfahren einen Aufreinigungsschritt aufweisen, nämlich die Aufreinigung des gebildeten Esters durch Abtrennung des Esters von im Permeat enthaltenden Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Das in Schritt c) erhaltene zu großen Teilen vom in Schritt a) gebildeten Ester befreite Permeat, welches mindestens nicht umgesetzte Alkohole und/oder nicht umgesetzte Kohlenwasserstoffe enthält, wird in einer bevorzugten Ausführungsform einer Recyclekomponentenabtrennung unterworfen. Dabei werden die nicht umgesetzten Alkohole und/oder nicht umgesetzten Kohlenwasserstoffe vom restlichen Permeat, insbesondere den dort enthaltenen Leichtsiedern, mittels thermischer Trennung, Extraktion, Kristallisation oder Membrantrennung abgetrennt. Bevorzugt wird ein thermisches Trennverfahren, besonders bevorzugt eine weitere Destillation eingesetzt, um die nicht umgesetzten Alkohole und/oder nicht umgesetzten Kohlenwasserstoffe vom restlichen Permeat abzutrennen. Die Verfahrensbedingungen sind dem Fachmann bekannt. Die dabei erhaltenen nicht umgesetzten Alkohole und/oder die nicht umgesetzten Kohlenwasserstoffe können dann in die Reaktionszone zurückgeführt werden.

Der nach dem erfindungsgemäßen Verfahren gebildete Ester kann in zwei weiteren nachfolgenden Verfahrensschritten d) und e) umgeestert werden. Dabei wird der Teil des Esters, der dem in Schritt a) eingesetzten ersten Alkohol entspricht, durch einen zweiten Alkohol ausgetauscht. Diese Umesterung wird nach dem oben genannten Schritt c), optional nach dem möglichen Aufreinigungsschritt, durchgeführt und umfasst die folgenden Schritte:

d) Umestern des in Schritt a) gebildeten Esters mit einem zweiten Alkohol, wobei dieser zweite Alkohol sich von dem im Schritt a) eingesetzten Alkohol unterscheidet, in einer zweiten Reaktionszone unter Erhalt eines zweiten Produktgemischs, welches zumindest den Ester mit dem zweiten Alkohol, den abgespaltenen ersten Alkohol und nicht umgesetzte zweite Alkohole umfasst;

e) Abtrennen des gebildeten Esters mit dem zweiten Alkohol vom restlichen zweiten Produktgemisch und insbesondere vom abgespaltenen ersten Alkohol mittels eines oder mehrerer Trennschritte, ausgewählt aus thermischer Trennung, Extraktion, Kristallisation oder Membrantrennung, vorzugsweise durch thermische Trennung und/oder mittels Membrantrennung, besonders bevorzugt mittels eines oder mehrerer Destillationsschritte und Rückführung des abgespaltenen ersten Alkohols in die Reaktionszone aus Schritt a), sowie Rückführung des nicht umgesetzten zweiten Alkohols in die zweite Reaktionszone.

In Schritt d) erfolgt die eigentliche Umesterung, also die Abspaltung des eigentlich in Schritt a) angebundenen ersten Alkohols und die Anbindung des zweiten Alkohols. Dazu wird der in Schritt a) gebildete Ester in einer Reaktionszone mit einem zweiten Alkohol, der sich vom ersten Alkohol unterscheidet, umgesetzt. In einer besonders bevorzugten Ausführungsform ist der bei der Umesterung eingesetzte zweite Alkohol im Vergleich zu dem in Schritt a) eingesetzten ersten Alkohol ein höhersiedender Alkohol. Der zweite Alkohol wird bei der Umesterung vorzugsweise im Überschuss zugegeben, um die Umesterungsreaktion zu begünstigen.

Der bei der Umesterung in Schritt d) eingesetzte zweite Alkohol ist vorzugsweise ein Mono- oder Polyalkohol (zwei oder mehr OH-Gruppen) mit 1 bis 50 Kohlenstoffatomen, weiterhin bevorzugt mit 1 bis 15 Kohlenstoffatomen, besonders bevorzugt mit 1 bis 10 Kohlenstoffatomen oder eine Mischung aus zwei oder mehr Mono- und/oder Polyalkoholen, mit der Maßgabe, dass der in Schritt a) eingesetzte erste Alkohol und der zweite Alkohol nicht identisch sind. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol, Pentaerythrit, Neopentylglykol, Trimethylolpropan, Dipentaerytriol oder Mischungen daraus, vorzugsweise Ethanol, 1-Propanol, 1-Butanol, 1-Pentanol, 1-Hexanol, 2-Propanol, tert-Butanol, 3-Pentanol, 2Propylheptanol.

Die Umesterung in Schritt d) wird vorzugsweise säure- oder basenkatalysiert durchgeführt. Als Säuren können Brönsted- oder Lewis-Säuren eingesetzt werden.

Geeignete Brönsted-Säuren für die Umesterung in Schritt d) sind Perchlorsäure, Schwefelsäure, Phosphorsäure, Methylphosphonsäure oder eine Sulfonsäure, beispielsweise Methansulfonsäure, Trifluormethansulfonsäure, tert-Butansulfonsäure, p-Toluolsulfonsäure(pTSA), 2-Hydroxypropan-2-sulfonsäure, 2,4,6-Trimethylbenzolsulfonsäure oder Dodecylsulfonsäure. Vorzugsweise werden Schwefelsäure oder eine Sulfonsäure als Brönsted-Säure eingesetzt, besonders bevorzugt Schwefelsäure. Es können auch Metall oder deren Verbindungen eingesetzt werden, beispielsweise Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat sowie Natriummethanolat und Kaliummethanolat.

Geeignete Lewis-Säuren für die Umesterung in Schritt d) sind Titan(IV)-isopropoxid, $Bu_2SnO$, BuSn(O)OH oder Aluminiumtriflat. Bevorzugt eingesetzte Lewis-Säuren sind Titan(IV)-isopropoxid, und Aluminiumtriflat

Geeignete Basen für die Umesterung in Schritt d) sind Alkalimetalle, Alkalialkoxide, Alkali- oder Erdalkaliacetate oder -oxide, Alkali- oder Erdalkalialkoholate, wie NaEtOH oder MgEtOH oder Alkalicarbonate, wie $K_2CO_3$ oder $Cs_2CO_3$. Es können aber auch basische Ionenaustauscher oder NaOH eingesetzt werden. Bevorzugt werden Na- oder Mg-Alkoholate wie NaEtOH oder MgEtOH eingesetzt.

Die säurekatalysierten Umesterung wird vorzugsweise bei einer Temperatur von 60 bis 220 °C, weiterhin bevorzugt von 100 bis 210 °C und besonders bevorzugt bei 130 bis 200 °C durchgeführt. Die Reaktion findet vorzugsweise oberhalb des Siedepunktes des abzuspaltenden ersten Alkohols statt, um den abgespaltenen ersten Alkohol direkt aus dem Reaktionsgemisch zu entfernen und somit eine Gleichgewichtsverlagerung zur Produktseite zu begünstigen. Der zweite Alkohol wird dabei vorzugsweise in einem signifikanten Überschuss, beispielsweise 30 : 1, zum in Schritt a) gebildeten Ester hinzugegeben.

Die basenkatalysierte Umesterung findet vorzugsweise bei einer Temperatur von 20 bis 100 °C statt.

Durch die beschriebe Umesterung wird ein zweites Produktgemisch erhalten, welches zumindest den Ester mit dem zweiten Alkohol, den abgespaltenen ersten Alkohol und nicht umgesetzte zweite Alkohole umfasst.

Der in Schritt d) gebildete zweite Ester wird im nachfolgenden Schritt e) vom restlichen zweiten Produktgemisch abgetrennt. Die Abtrennung wird mittels thermischer Trennung, vorzugsweise Destillation, und/oder mittels Membrantrennung, insbesondere mit den oben beschriebenen Membranmaterialien, durchgeführt. Die entsprechenden Verfahrensbedingungen sind dem Fachmann bekannt.

Es kann vorkommen, dass bei dem in Schritt e) verwendeten Trennverfahren, insbesondere bei der Destillation, nicht nur der gebildete Ester vom restlichen zweiten Produktgemisch abgetrennt wird, sondern auch möglicherweise gebildete Hochsieder, beispielsweise hochsiedende Nebenprodukte, die bei der Umsetzung in Schritt c1) anfallen können. Um diese Hochsieder zu entfernen kann das erfindungsgemäße Verfahren einen Aufreinigungsschritt aufweisen, nämlich die Aufreinigung des in Schritt c1) gebildeten Esters durch Abtrennung des Esters von den vorhandenen Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Die erfindungsgemäß erhaltenen Ester können als Edukte zur Herstellung weiterer Produkte dienen. Wichtige Reaktionen der erhaltenen Ester sind die Hydrierung zur Bildung von Alkoholen, die Hydrolyse bzw. Verseifung zur Bildung von Carbonsäuren bzw. Carbonsäuresalzen oder die Aminolyse zur Bildung von Amiden.

Beispiele:

MET Zellen (Batch-Fahrweise):

Die Versuche wurden in einer kommerziell erhältlichen Dead-End Batch-Filtrationszelle des Typs METcell von Evonik MET mit den in Tabelle 1-3 gelisteten kommerziell erhältlichen Membranen und der erfindungsgemäßen PEEK-Aceton Membran durchgeführt. Die erfindungsgemäße PEEK-Aceton Membran wurde analog zur Publikation J. da Silva Burgal et al.; Journal of Membrane Science, Vol. 479 (2015), S. 105-116 (s. auch WO 2015/110843 A1) hergestellt.

Versuche wurden bei den folgenden Bedingungen gefahren: 56,7 $cm^2$ aktive Membranfläche, 20 bar Transmembrandruck, 25 °C Separationstemperatur (bei PEEK 50 °C), 250 U/min Rührergeschwindigkeit.

Zur Bestimmung des Rückhalts wurde der Marker 4,4'-Di-tert-butyl-2,2'-dipyridyl mit 0,01 Gew.-% dem Untersuchungsgemisch hinzugefügt. Der angegebene Marker wurde so ausgewählt, dass die Struktur und die molekulare Masse repräsentativ ist für übliche in der Methoxycarbonylierung eingesetzten Liganden (z. B. EP 3 121 186 A2) und in der geringen Konzentration katalytischen Mengen entspricht. Zusätzlich wurden 0,5 Gew.-% Aluminiumtriflat (bezogen auf das Gesamtgewicht der Mischung) als Säure hinzugegeben.

Die METcell wurde mit 200 ml der vorgenannt beschriebenen Mischung befüllt und auf Betriebstemperatur (= Separationstemperatur) gebracht. Anschließend wurde Stickstoff bis zu einem Betriebsdruck von 20 bar aufgedrückt. Insgesamt wurden 100 ml aus der METcell als Permeat entnommen, wobei das Gewicht des Permeats kontinuierlich erfasst wurde. Zum Ende des Versuchs, also nachdem 100 ml Permeat aus der METcell entnommen worden sind, wurden Proben aus Retentat und Permeat für GC und HPLC Analytik gezogen. Die angegebene Permeabilität ist ein Mittelwert über die 100 ml gesammeltes Permeat (Ergebnisse siehe Tabelle 1).

Der Rückhalt wurde per HPLC-UV auf einer C18-Säule (Ergebnisse siehe Tabelle 2) und Alkoholselektivität per GC-FID (Ergebnisse siehe Tabelle 3) bestimmt. Die Verfahren sind dem Fachmann bekannt.

Beispiel 1

Es wurden unterschiedliche Membranmaterialien mit einer Mischung untersucht bestehend aus Methanol mit 43 Gew.-% und Methyloctanoat mit 57 Gew.-% was einem molaren Verhältnis von 4:1 entspricht. Zusätzlich waren der Marker (0,01 Gew.-% 4,4'-Di-tert-butyl-2,2'-dipyridyl) und die Säure (0,5 Gew.-% Aluminiumtriflat) wie erwähnt in der Mischung vorhanden.

Tabelle 1: Vergleich der Permeabilität

| Membran | $P_{t=0}$ | $P_{t=70h}$ | $P_{t=300h}$ | $P_{rel\,t=70h}$ | $P_{rel\,t=300h}$ |
|---|---|---|---|---|---|
| | kg/ $m^2h^1bar^1$ | | | % | |
| GMT oNF-2 | 0,6 | 1,63 | -* | 272 | -* |
| Evonik DuraMem 300 | 0,09 | ** | 0,44 | ** | 489 |
| Evonik PuraMem S | 0,68 | 1,95 | 10,65 | 287 | 1566 |
| PEEK | 0,23 | 0,27 | 0,28 | 117 | 122 |
| Solsep NF 010206 S | 0,32 | 0,70 | 0,84 | 219 | 263 |
| Solsep NF 030306 F | 0,15 | 0,31 | 0,38 | 207 | 253 |
| * Kein Datenpunkt aufgrund von Materialversagen ** Datenpunkt liegt nicht vor | | | | | |

Tabelle 2: Vergleich des Membranrückhaltes (für den Marker)

| Membran | $R_{70h}$ | $R_{150h}$ | |
|---|---|---|---|
| | % | | |
| GMT oNF-2 | 97 | 94 | * |
| Evonik DuraMem 300 | 64 | ** | 59 |
| Evonik PuraMem S | 78 | 40 | 27 |
| PEEK | 81*** | 77 | 80 |
| Solsep NF 010206 S | 39 | 24 | 5 |
| Solsep NF 030306 F | 90 | 60 | 59 |
| * Kein Datenpunkt aufgrund von Materialversagen ** Datenpunkt liegt nicht vor *** gemessen bei 22h | | | |

Tabelle 3: Vergleich der Alkoholselektivitäten (hier für Methanol)

| | S für Methanol | | |
|---|---|---|---|
| Membran | $S_{70h}$ | $S_{150h}$ | $S_{300h}$ |
| GMT oNF-2 | 1,38 | 1,36 | -* |
| Evonik DuraMem 300 | 1,04 | ** | 1,18 |
| Evonik PuraMem S | 0,93 | 0,96 | 0,92 |
| PEEK | 0,81 | 0,72 | 0,71 |
| Solsep NF 010206 S | 1,1 | 1,09 | 1,00 |

(fortgesetzt)

| Membran | S für Methanol | | |
|---|---|---|---|
| | $S_{70h}$ | $S_{150h}$ | $S_{300h}$ |
| Solsep NF 030306 F | 1,23 | 1,22 | 1,17 |
| * Kein Datenpunkt aufgrund von Materialversagen ** Datenpunkt liegt nicht vor | | | |

Den Tabellen ist zu entnehmen, dass die Vergleichsbeispiele der Membranen DuraMem 300 und PuraMem S mit der Zeit einen zum Teil extremen Anstieg der Permeabilität zeigen. Bei der PuraMem S wurde anhand des Membranrückhalts festgestellt, dass das Membranmaterial mit der Zeit seine technische Integrität verloren hat. Dagegen zeigte die DuraMem 300 relativ konstante Werte für den Rückhalt, jedoch nach 300 Stunden einen inakzeptablen Anstieg der Permeabilität und war dann auch nur noch wenig selektiv für das Zurückhalten von Methanol. Die Membran GMT oNF-2 hat keine 300 Stunden ausgehalten, sondern wurde aufgrund mangelnder Säurestabilität zerstört. Die Membran Solsep NF 010206 S zeigte bereits nach 70 Stunden einen inakzeptablen Anstieg der Permeabilität und zeigte auch sonst keine besondere Selektivität (~1) für Methanol. Die Membran Solsep NF 030306 F zeigte Werte >1 für die Selektivität von Methanol und zeigt demnach keine Selektivität für Methanol. Weiterhin wurde bei dieser Membran ein inakzeptabler Anstieg der Permeabilität festgestellt.

Die erfindungsgemäße Membran PEEK-Aceton zeigt dagegen auch nach 300 Stunden eine gute Permeabilität mit nur geringem Anstieg, einen guten Rückhalt und eine gute Selektivität (<1) von Methanol. Methanol wird also auch im Retentat angereichert.

Beispiel 2

Es wurden PEEK-Membranen mit Mischungen unterschiedlicher Alkohole und Ester untersucht. Zusätzlich waren der Marker (0,01 Gew.-% 4,4'-Di-tert-butyl-2,2'-dipyridyl) und die Säure (0,5 Gew.-% Aluminiumtriflat) wie bereits in Beispiel 1 erwähnt in der Mischung vorhanden. Ester und Alkohole wurden analog zu Beispiel 1 in einem solchen molaren Verhältnis eingesetzt, dass immer etwa 4 Alkoholgruppen pro Estergruppe vorhanden sind, d. h. das molare Verhältnis jeweils an die Anzahl der funktionellen Gruppen angepasst wurde. Die Untersuchungen wurden mit den Estern Methylpropionat, Isopropylproprionat, Methyltetradecanoat, Octylbutyrat, Octyloctanoat, Octylisovalerat und Benzylpropionat, dem Diester Dimethyladipat und den Alkoholen Methanol, Isopropanol, Octanol, Benzylalkohol und 2-Ethylhexanol durchgeführt. Die Ester wurden so gewählt, dass sie aus Olefinen unterschiedlicher Kettenlänge und Anzahl olefinischer Doppelbindungen bestehen. Zudem wird der Ester hinsichtlich potenzieller Alkohole (C1-C8) variiert. Die Versuche wurden bei den folgenden Bedingungen gefahren: 56,7 cm$^2$ aktive Membranfläche, 40 bar Transmembrandruck, 50 °C Separationstemperatur, 250 U/min Rührergeschwindigkeit.

Tabelle 4: Vergleich der Alkoholselektivitäten unter Verwendung von PEEK-Membranen

| Gemisch Alkohol / Ester | S für den verwendeten Alkohol | | |
|---|---|---|---|
| | $S_{0h}$ | $S_{150h}$ | $S_{300h}$ |
| Methylpropionat / Methanol | 0,93 | 0,92 | 0,94 |
| Isopropylpropionat / Isopropanol | 0,93 | 0,91 | 0,93 |
| Methyltetradecanoat / Methanol | 1,01 | 0,86 | ** |
| Octylbutyrat / Octanol | 0,92 | 0,92 | 0,95 |
| Octyloctanoat / Octanol | 0,92 | 0,88 | 0,93 |
| Octylisovalerat / Octanol | 0,95 | 0,94 | 0,94 |
| Benzylpropionat / Benzylalkohol | 0,98 | 0,99 | 0,99 |
| Dimethyladipat / Methanol | 0,98 | ** | 0,97 |
| ** kein Datenpunkt vorhanden | | | |

Es zeigt sich, dass auch bei der Verwendung unterschiedlicher Alkohole und unterschiedlicher Ester eine gute Selektivität für die eingesetzten Alkohole erreicht werden kann.

Tabelle 5: Vergleich des Membranrückhaltes (für den Marker) unter Verwendung von PEEK-Membranen

| Gemisch Alkohol / Ester | $R_{0h}$ | $R_{150h}$ | $R_{300h}$ |
|---|---|---|---|
| | % | | |
| Methylpropionat / Methanol | 97 | 97 | 97 |
| Isopropylpropionat / Isopropanol | 99 | 99 | 99 |
| Methyltetradecanoat / Methanol | 86 | 85 | ** |
| Octylbutyrat / Octanol | 99,9 | 97 | 97 |
| Octyloctanoat / Octanol | 95 | 97 | 97 |
| Octylisovalerat / Octanol | *** | *** | *** |
| Benzylpropionat / Benzylalkohol | *** | *** | *** |
| Dimethyladipat / Methanol | 89 | ** | 88 |
| ** Datenpunkt nicht vorhanden *** Datenpunkt noch nicht vorhanden | | | |

Es zeigt sich, dass auch hinsichtlich des Membranrückhalts für den Marker bei allen verwendeten Gemischen aus Alkohol und Ester über die Zeit gute Ergebnisse erzielt werden konnten.

Tabelle 6: Vergleich der Permeabilität unter Verwendung von PEEK-Membranen

| Gemisch Alkohol / Ester | $P_{t=0}$ | $P_{t=150h}$ | $P_{t=300h}$ |
|---|---|---|---|
| | $L / m^2 h^1 bar^1$ | | |
| Methylpropionat / Methanol | 0,065 | 0,063 | 0,063 |
| Isopropylpropionat / Isopropanol | 0,031 | 0,033 | 0,036 |
| Methyltetradecanoat / Methanol | 0,035 | 0,054 | ** |
| Octylbutyrat / Octanol | 0,008 | 0,008 | 0,010 |
| Octyloctanoat / Octanol | 0,005 | 0,006 | 0,009 |
| Octylisovalerat / Octanol | 0,014 | 0,016 | 0,015 |
| Benzylpropionat / Benzylalkohol | 0,199 | 0,204 | 0,168 |
| Dimethyladipat / Methanol | 0,026 | ** | 0,028 |
| ** Datenpunkt nicht vorhanden | | | |

Die Permeabilitäten sind zum Teil sehr gering, was beispielsweise an den längeren Kohlenstoffatomketten oder allgemein der Größe der Moleküle liegt. Trotz der geringen Permeabilität konnten gute Rückhalte (für den Marker) gute Selektivitäten (für Methanol) erreicht werden. Weiterhin ist der Tabelle zu entnehmen, dass die Permeabilität über 300 h nicht erheblich steigt.

Beispiel 3: 3er-Teststand (Kontinuierliche-Fahrweise)

Die Versuche wurden in einer durchgehend betriebenen Versuchsanlage mit vollständiger Rückführung des Permeats im geschlossenen Kreislauf durchgeführt. Im Kern besteht die Anlage aus einem auf bis zu 60 bar bedrückbaren, überströmten Hochdruckkreislauf mit drei Flachkanal-Membranzellen. Der Kreislauf wird aus einer mit Feedlösung gefüllten, mechanisch durchmischten und mit Stickstoff überschleierten Vorlage mit Fassungsvolumen von 5 L gespeist. Mittels Membrankolbenpumpe wird die Feedlösung auf den Betriebsdruck des Membrankreislaufs und somit in den Hochdruckbereich der Versuchsanlage gebracht werden. Der Hochdruckbereich der Versuchsanlage besteht im Wesentlichen aus einem Flüssigkeitskreislauf, der über eine Kreislaufpumpe betrieben wird, und drei Flachmembrantestzellen sowie der benötigten Sensorik (z.B. Druckmessung, Messung der Überströmmenge im Kreislauf). Aus den Membranmodulen wird der durch die Membran dringende Flüssigkeitsstrom als Permeat abgeführt und wieder der Vorlage zugeführt. Die Permeatmenge wird per Coriolis-Messprinzip für alle drei Membranen kontinuierlich erfasst. Der Hochdruckkreislauf wird über eine Kreiselpumpe umgewälzt, um die erforderliche Überströmung der Membranen zu gewährleisten. Die Überströmung wird gewöhnlich so hoch gewählt, dass die Konzentrationen auf Feed- und Retentatseite der Membran näherungsweise gleich sind. (Dies ist der Fall, wenn die Überströmmenge den ausgeschleusten Permeatstrom um ein Weites übersteigt.) Auch die Feedzufuhr in den Hochdruckkreislauf über die HD-Pumpe wird so gewählt, dass der Volumenstrom des Zustroms den Volumenstrom des Permeats um ein Vielfaches übersteigt. Die überschüssige Feedmenge (Förderstrom der Hochdruckpumpe minus Summe des Permeats der drei Membranen) wird ebenfalls zurück in die Vorlage geführt. Diese Rückführung erfolgt über einen mechanischen Vordruckregler, mit dem auch der Vordruck der Nanofiltrationsstufe eingestellt wird. Der Kreislauf wird durch einen Thermostaten beheizt, sodass eine definierte Temperatur für die Trennung gewährleistet wird. Die Versuche wurden bei den folgenden Bedingungen gefahren: 84,5 $cm^2$ aktive Membranfläche pro Modul, 20 bar Transmembrandruck, 40 °C Separationstemperatur.

Es wurden unterschiedliche Membranmaterialien mit einer Mischung aus 43 Gew.-% Methanol und 57 Gew.-% Methyloctanoat (molares Verhältnis 4:1) untersucht. Zur Bestimmung des Rückhalts wurde wie in den vorherigen Beispielen der Marker 4,4'-Di-tert-butyl-2,2'-dipyridyl mit 0,01 Gew.-% dem Untersuchungsgemisch hinzugefügt. Zusätzlich wurden 0,5 Gew.-% Aluminiumtriflat (bezogen auf das Gesamtgewicht der Mischung) nach einer definierten Betriebszeit hinzugegeben. Der Zeitpunkt der Säurezugabe wird in nachfolgenden Tabellen 7 bis 9 als Zeitpunkt t=0h definiert. Zu den definierten Beprobungszeiten wurden Flüssigkeitsproben der jeweiligen Permeatströme, des Feeds und des Retentats entnommen.

Die Permeabilität wurde wie vorgenannt bestimmt (Ergebnisse in Tabelle 7). Der Markerrückhalt wurde per HPLC mit UV-Detektor (260 nm) auf einer $C_{18}$-Säule bestimmt (Ergebnisse in Tabelle 8) und die Alkoholselektivität wurde per GC mit FID-Detektor bestimmt (Ergebnisse in Tabelle 9).

Tabelle 7: Vergleich der Permeabilität

| Membran | $P_{t=0h}$ | $P_{t=100h}$ | $Prel_{t=100h}$ |
|---|---|---|---|
| | $L/m^2h^1bar^1$ | | % |
| PEEK | 0,21 | 0,21 | 100 |
| GMT oNF2 | 5,59 | * | * |
| SolSep 030306F | 0,59 | 1,36 | 244 |
| SolSep NF030306 | 0,79 | 0,96 | 120 |
| SolSep NF030705 | 1,86 | 6,96 | 377 |
| SolSep NF030105 | 2,58 | 8,22 | 329 |
| * Kein Datenpunkt aufgrund von Materialversagen | | | |

Tabelle 8: Vergleich des Membranrückhaltes

| Membran | $R_{t=50h}$ | $R_{t=100h}$ |
|---|---|---|
| | % | |
| PEEK | 93,3 | 93,5 |
| GMT oNF2 | * | * |
| SolSep 030306F | 41,3 | 45,3 |
| SolSep NF030306 | 62,5 | 67,7 |
| SolSep NF030705 | 14 | 14,7 |
| SolSep NF030105 | 49,9 | 56,3 |
| * Kein Datenpunkt aufgrund von Materialversagen | | |

Tabelle 9: Vergleich der Alkoholselektivitäten (hier für Methanol)

| | S für Methanol | |
|---|---|---|
| | % | |
| Membran | $S_{t=50h}$ | $S_{t=100h}$ |
| PEEK | 0,9 | 0,9 |
| GMT oNF2 | * | * |
| SolSep 030306F | 1,09 | 1,11 |
| SolSep NF030306 | 1,16 | 1,19 |
| SolSep NF030705 | 1,03 | 1,05 |
| SolSep NF030105 | 1,05 | 1,08 |
| * Kein Datenpunkt aufgrund von Materialversagen | | |

Die Membran GMT oNF-2 hat keine 300 Stunden ausgehalten, sondern wurde aufgrund mangelnder Säurestabilität zerstört. Alle Solsep-Membranen zeigten bereits nach 100 Stunden einen inakzeptablen Anstieg der Permeabilität und zeigten auch sonst keine besondere Selektivität für Methanol, sondern Werte >1 für die Selektivität von Methanol, was bedeutet, dass Methanol bevorzugt durch die Membran durchtritt und nicht zurückgehalten wird. Die erfindungsgemäße Aufgabe konnte mit den Solsep-Membranen daher nicht gelöst werden, Die erfindungsgemäße PEEK-Membran zeigt dagegen auch nach 100 Stunden eine gute Permeabilität, einen guten Rückhalt und eine gute Selektivität (<1) für Methanol. Methanol wird also auch im Retentat angereichert.

Beispiel 4

Umsetzung von Diisobuten (DiB) zu Methyl-3,5,5-trimethylhexanoat (TMH-Ester) mit Membranabtrennung als Katalysator-Recycling. DiB stellt ein Gemisch bestehend aus den beiden C8-Isomeren 2,4,4-Trimethylpent-1-en und 2,4,4-Trimethylpent-2-en in den Verhältnissen von ungefähr 80:20 dar. Die Versuche wurden in einer durchgehend betriebenen Versuchsanlage durchgeführt, die wie folgt aufgebaut ist.

Im Kern besteht die Anlage aus einem 200 mL Glasautoklav (= Reaktor) der Firma Büchi (bis zu 10 bar bedrückbar). Der Autoklav wird aus einer mit Reaktionslösung gefüllten und mit Argon überschleierten Glasvorlage mittels HPLC Pumpen der Firma Knauer gespeist. Eine weitere HPLC Pumpe der Firma Knauer pumpt aus dem Glasautoklav in den separaten auf bis zu 60 bar bedrückbaren Hochdruckkreislauf, Der Hochdruckkreislauf der Versuchsanlage besteht im Wesentlichen aus einem Flüssigkeitskreislauf, der über eine Kreislaufpumpe betrieben wird, und einer Flachmembrantestzellen sowie der benötigten Sensorik (z.B. Druckmessung, Messung der Temperatur). Der Hochdruckkreislauf wird über eine Kreiselpumpe umgewälzt, um die erforderliche Überströmung der Membranen zu gewährleisten. Aus den Membranmodul wird der durch die Membran dringende Flüssigkeitsstrom als Permeat abgeführt und in einer mit Argon überschleierten Glasvorlagen aufgefangen. Die Permeatmenge wird mittels Waage kontinuierlich erfasst. Die überschüssige Feedmenge (Retentat) wird zurück in den Glasautoklaven geführt. Diese Rückführung erfolgt über einen mechanischen Vordruckregler, mit dem auch der Vordruck der Nanofiltrationsstufe eingestellt wird. Der Kreislauf wird durch einen Thermostaten beheizt, sodass eine definierte Temperatur für die Trennung gewährleistet wird. Die Versuche wurden bei den folgenden Bedingungen gefahren: 84,5 cm$^2$ aktive Membranfläche, 45 bar Transmembrandruck, 25 °C Separationstemperatur.

Proben können sowohl am Feedstrom zum Hochdruckkreislauf (Feed), aus dem Hochdruckkreislauf (Retentat) und vom Permeat gezogen werden. Zur Analyse der Ausbeute wurden 0,08g der Probe und 0,03g Ethylbenzol (interner Standard) eingewogen und mit 0,25g Acetonitril verdünnt. Die weitere Analyse erfolgte mittels GC-FID. Der Rückhalt an Liganden und Metall wurde Mittels ICP-OES nach vorherigem Aufschluss der Proben von Permeat und Retentat ermittelt. Die Analyse bezüglich des Rückhalts an Säure erfolgte mittels $^{19}$F-NMR.

Die Probenvorbereitung und Analyse alle drei Methoden sind dem Fachmann bekannt.

Als Membranmaterial wurde PEEK eingesetzt. Die erfindungsgemäße PEEK-Aceton Membran wurde analog zur Publikation J. da Silva Burgal et al.; Journal of Membrane Science, Vol. 479 (2015), S. 105-116 (s. auch WO 2015/110843 A1) hergestellt.

Der Inhalt des Glasautoklav wurde gerührt, um eine nahezu ideale Durchmischung zu gewährleisten. Der Druck im Glasautoklav wurde über einen mechanischen Vordruckregler auf 10 bar eingestellt. Der Glasautoklav wird über ein Ölbad mit einer externen im Glasautoklaven angebrachten Temperaturmessung auf der gewünschten Temperatur gehalten.

Zunächst wurde der Glasautoklav und der Hochdruckkreislauf mit einer Grundreaktionsmasse bestehend aus 56 Gew.-% MeOH, 40 Gew.-% DiB, 0,5 Gew.-% 1,2-bis((tert-butyl(pyridin-2-yl)phosphanyl)methyl)benzen (Ligand), 3,4 Gew.-% Aluminiumtriflat (Säure) und 0,1 Gew.-% [Pd(acac)$_2$] (Metall) gefüllt und mit 10bar Argon inertisiert und in Betrieb genommen.

Der Glasautoklav wurde dann auf 100°C Innentemperatur aufgeheizt und der anfängliche Argonstrom auf CO geändert (Reaktionsstart). Über 15 h wurde die Grundreaktionsmasse zum Anfahren des Systems im Kreis gepumpt. Nach 15 h wurde DiB im Feed zu 99% zum TMH-Ester umgesetzt. Nach 15h wurde die Kreis-Fahrweise beendet und das Permeat in separaten Glasbehältern aufgefangen und frische Reaktionslösung (60 Gew.-% MeOH und 40 Gew.-% DiB) zudosiert. Für die in Tabelle 1 gelisteten Versuchszeiträume wurden neue Permeat-Auffangbehälter genutzt. Frischer Katalysator, Ligand oder Säure wurde über den Untersuchungszeitraum von 105h nicht hinzugegeben. Es wurden für die jeweiligen Untersuchungszeiträume die Permeabilität, die Ausbeute des TMH-Esters und die Rückhalte für die Komponenten des Katalysatorsystems mit den oben genannten Methoden bestimmt.

Tabelle 1 Kontinuierliche Umsetzung von DiB zu TMH-Ester

| Zeitraum | Ti(Glasautoklav) | P | Y(TMH-Ester) * | R(Metall) | R(Ligand) | R(Säure) |
|---|---|---|---|---|---|---|
| h | °C | kg/m$^2$h$^1$bar$^1$ | % | % | % | % |
| 0-15 | 100 | 0,045 | 99 | ** | | |
| 15-35 | 100 | 0,051 | 84 | 98 | 97 | 97 |
| 35-70 | 90 | 0,049 | 73 | 97 | 98 | 96 |
| 70-90 | 80 | 0,050 | 59 | 98 | 97 | 97 |
| 90-105 | 100 | 0,048 | 84 | 97 | 98 | 96 |
| * Ausbeute zum Ende des jeweils betrachteten Versuchszeitraums | | | | | | |
| ** Keine Rückhalte ermittelt (Anfahren des Systems). | | | | | | |

## Patentansprüche

1. Verfahren zur Herstellung eines Esters durch Carbonylierung eines C2- bis C16-Kohlenwasserstoffs, wobei das Verfahren die folgenden Schritte umfasst:

   a) Umsetzen (Carbonylieren) eines C2- bis C16-Kohlenwasserstoffs mit mindestens einer olefinischen Doppelbindung mit Kohlenmonoxid und mit einem Alkohol, welcher ein Mono- oder Polyalkohol (zwei oder mehr OH-Gruppen) mit 1 bis 50 Kohlenstoffatomen oder eine Mischung aus zwei oder mehr Mono- und/oder Polyalkoholen ist und welcher, wenn es sich um einen Monoalkohol handelt, in einem Molverhältnis zum C2- bis C16-Kohlenwasserstoff (Alkohol: C2- bis C16-Kohlenwasserstoff) von 2 bis 20 eingesetzt wird oder welcher, wenn es sich um einen Polyalkohol handelt, in einem Molverhältnis zum eingesetzten Kohlenwasserstoff (Kohlenwasserstoff: Polyalkohol) von 2 bis 20 eingesetzt wird, in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer Reaktionszone unter Erhalt eines flüssigen Produktgemisches, welches zumindest den durch die Umsetzung gebildeten Ester, das homogene Katalysatorsystem, Leichtsieder, Hochsieder und nicht umgesetzte Alkohole umfasst;

   b) Durchführen einer Membrantrennung zur Abtrennung des homogenen Katalysatorsystems aus dem flüssigen Produktgemisch, wodurch das homogene Katalysatorsystem und zusätzlich nicht umgesetzter Kohlenwasserstoff und/oder nicht umgesetzter Alkohol, vorzugsweise nicht umgesetzter Alkohol im Retentat angereichert werden und der in Schritt a) gebildete Ester im Permeat angereichert wird, wobei als Membranmaterial ein säurestabiles, d. h. ein mindestens 300 h in Anwesenheit der Säure des Katalysatorsystems stabiles OSN-fähiges (Organic Solvent Nanofiltration) Membranmaterial, welches zumindest eine trennaktive Schicht aufweist, die aus PEEK (Polyetheretherketon) besteht, eingesetzt wird, und Rückführung des Retentats in die Reaktionszone;

   c) Aufarbeitung des Permeat mittels eines oder mehrerer Trennschritte, ausgewählt aus thermischer Trennung, Extraktion, Kristallisation oder Membrantrennung, vorzugsweise mittels eines oder mehrerer Destillationsschritte, zur Abtrennung des in Schritt a) gebildeten Esters und zur Abtrennung des nicht umgesetzten C2- bis C16-Kohlenwasserstoffs mit mindestens einer olefinischen Doppelbindung und/oder des nicht umgesetzten Alkohols und Rückführung des nicht umgesetzten C2- bis C16-Kohlenwasserstoffs mit mindestens einer olefinischen Doppelbindung und/oder des nicht umgesetzten Alkohols in die Reaktionszone von Schritt a).

2. Verfahren nach Anspruch 1, wobei das erfindungsgemäße Membranmaterial nach einem Einsatz im hier beschriebenen Membrantrennverfahren in Schritt b) von mindestens 300 Stunden die folgenden beiden Merkmale aufweist:

• der Membranrückhalt R für den Ligand des homogene Katalysatorsystems beträgt mindestens 75%, und verschlechtert sich um nicht mehr als 30 Prozentpunkte; oder
• die relative Permeabilität $P_{rel}$ weist einen Wert zwischen 30% und 300% auf.

3. Verfahren nach Anspruch 1 oder 2, wobei der in Schritt a) eingesetzte Alkohol ein Mono- oder Polyalkohol (zwei oder mehr OH-Gruppen) mit 1 bis 15 Kohlenstoffatomen oder eine Mischung aus zwei oder mehr Mono- und/oder Polyalkoholen ist.

4. Verfahren nach Anspruch 3, wobei der in Schritt a) eingesetzte Alkohol aus der Gruppe, bestehend aus Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol und Mischungen daraus, ausgewählt wird.

5. Verfahren nach einem der vorhergehenden Ansprüche, wobei der in Schritt a) eingesetzte C2- bis C16-Kohlenwasserstoff mit mindestens einer olefinischen Doppelbindung ein n- oder iso-Alken mit 2 bis 16 Kohlenstoffatomen ist.

6. Verfahren nach einem der vorhergehenden Ansprüche, wobei das Metall der Gruppe 8 bis 10 des Periodensystems der Elemente oder eine Verbindung davon des homogenen Katalysatorsystems in Schritt a) Palladium oder eine Verbindung davon ist.

7. Verfahren nach einem der vorhergehenden Ansprüche, wobei der phosphorhaltige Ligand des homogenen Katalysatorsystems eine Bidentat-Struktur aufweist.

8. Verfahren nach einem der vorhergehenden Ansprüche, wobei die Säure des Katalysatorsystems in Schritt a) eine Brönsted-Säure mit einem pKs ≤ 5oder eine Lewis-Säure mit einem LAU-Wert von mehr als 25 ist.

9. Verfahren nach einem der vorhergehenden Ansprüche, wobei die Säure des Katalysatorsystems in Schritt a) eine Brönsted-Säure oder eine Lewis-Säure ist, wobei die Brönsted-Säure Perchlorsäure, Schwefelsäure, Phosphorsäure, Methylphosphonsäure oder eine Sulfonsäure ist und wobei die Lewis-Säure Aluminiumtriflat, Aluminiumchlorid, Aluminiumhydrid, Trimethylaluminium, Tris(pentafluorophenyl)boran, Bortrifluorid, Bortrichlorid oder eine Mischungen davon ist.

10. Verfahren nach einem der vorhergehenden Ansprüche, wobei die Reaktionszone für die Umsetzung in Schritt a) mindestens einen Reaktor aufweist.

11. Verfahren nach einem der vorhergehenden Ansprüche, wobei das Verfahren nach der Abtrennung in Schritt c) die folgenden weiteren Schritte aufweist:

d) Umestern des in Schritt a) gebildeten Esters mit einem zweiten Alkohol, wobei dieser zweite Alkohol sich von dem im Schritt a) eingesetzten Alkohol unterscheidet, in einer zweiten Reaktionszone unter Erhalt eines zweiten Produktgemischs, welches zumindest den Ester mit dem zweiten Alkohol, den abgespaltenen ersten Alkohol und nicht umgesetzte zweite Alkohole umfasst, wobei der zweite Alkohol in äquimolarer Menge oder im Überschuss eingesetzt wird;
e) Abtrennen des gebildeten Esters mit dem zweiten Alkohol vom restlichen zweiten Produktgemisch und insbesondere vom abgespaltenen ersten Alkohol mittels eines oder mehrerer Trennschritte, ausgewählt aus thermischer Trennung, Extraktion, Kristallisation oder Membrantrennung und Rückführung des abgespaltenen ersten Alkohols in die Reaktionszone aus Schritt a), sowie Rückführung des nicht umgesetzten zweiten Alkohols in die zweite Reaktionszone.

12. Verfahren nach Anspruch 11, wobei der in Schritt e) eingesetzte zweite Alkohol im Vergleich zum ersten Alkohol ein höhersiedender Alkohol ist.

13. Verfahren nach Anspruch 11 oder 12, wobei ein Teil der abgespaltenen ersten Alkohole bereits aus der zweiten Reaktionszone entnommen und zur ersten Reaktionszone zurückgeführt wird.

**Claims**

1. Process for preparing an ester by carbonylation of a C2 to C16 hydrocarbon, with the process comprising the following steps:

   a) reacting (carbonylating) a C2 to C16 hydrocarbon having at least one olefinic double bond with carbon monoxide and with an alcohol that is a mono- or polyol (two or more OH groups) having 1 to 50 carbon atoms or is a mixture of two or more mono- and/or polyols and which, when it is a monool, is used in a molar ratio to the C2 to C16 hydrocarbon (alcohol:C2 to C16 hydrocarbon) of 2 to 20 or which, when it is a polyol, is used in a molar ratio to the hydrocarbon used (hydrocarbon:polyol) of 2 to 20, in the presence of a homogeneous catalyst system comprising at least one metal of groups 8 to 10 of the periodic table of the elements or a compound thereof, a phosphorus-containing ligand and an acid, in a reaction zone to obtain a liquid product mixture that comprises at least the ester formed by the reaction, the homogeneous catalyst system, low boilers, high boilers and unreacted alcohols;
   b) carrying out a membrane separation to separate the homogeneous catalyst system from the liquid product mixture, whereby the homogeneous catalyst system and additionally unreacted hydrocarbon and/or unreacted alcohol, preferably unreacted alcohol, are enriched in the retentate and the ester formed in step a) is enriched in the permeate, wherein the membrane material used is an OSN (organic solvent nanofiltration) membrane material that is acid-stable, i.e. stable in the presence of the acid of the catalyst system for at least 300 h, and has at least one separation-active layer, which is composed of PEEK (polyether ether ketone), and the retentate is recycled into the reaction zone;
   c) working up the permeate by means of one or more separation steps selected from thermal separation, extraction, crystallization or membrane separation, preferably by means of one or more distillation steps, for removing the ester formed in step a) and for removing the unreacted C2 to C16 hydrocarbon having at least one olefinic double bond and/or the unreacted alcohol and recycling of the unreacted C2 to C16 hydrocarbon having at least one olefinic double bond and/or of the unreacted alcohol into the reaction zone of step a).

2. Process according to Claim 1, wherein, after being used for at least 300 hours in the membrane separation process in step b) described herein, the membrane material according to the invention exhibits the following two characteristics:

   • the membrane retention R for the ligand of the homogeneous catalyst system is at least 75% and worsens by not more than 30 percentage points; or
   • the relative permeability $P_{rel}$ has a value between 30% and 300%.

3. Process according to Claim 1 or 2, wherein the alcohol used in step a) is a mono- or polyol (two or more OH groups) having 1 to 15 carbon atoms or a mixture of two or more mono- and/or polyols.

4. Process according to Claim 3, wherein the alcohol used in step a) is selected from the group consisting of methanol, ethanol, 1-propanol, 1-butanol, 1-pentanol, 1-hexanol, 2-propanol, tert-butanol, 3-pentanol, 2-propylheptanol, cyclohexanol, phenol and mixtures thereof.

5. Process according to any of the preceding claims, wherein the C2 to C16 hydrocarbon having at least one olefinic double bond that is used in step a) is an n- or isoalkene having 2 to 16 carbon atoms.

6. Process according to any of the preceding claims, wherein the metal of groups 8 to 10 of the periodic table of the elements or a compound thereof in the homogeneous catalyst system in step a) is palladium or a compound thereof.

7. Process according to any of the preceding claims, wherein the phosphorus ligand in the homogeneous catalyst system has a bidentate structure.

8. Process according to any of the preceding claims, wherein the acid in the catalyst system in step a) is a Brønsted acid having a pKa $\leq$ 5 or a Lewis acid having an LAU value of more than 25.

9. Process according to any of the preceding claims, wherein the acid in the catalyst system in step a) is a Brønsted acid or a Lewis acid, the Brønsted acid being perchloric acid, sulfuric acid, phosphoric acid, methylphosphonic acid or a sulfonic acid, and the Lewis acid being aluminium triflate, aluminium chloride, aluminium hydride, trimethylaluminium, tris(pentafluorophenyl)borane, boron trifluoride, boron trichloride or a mixture thereof.

10. Process according to any of the preceding claims, wherein the reaction zone for the reaction in step a) includes at least one reactor.

11. Process according to any of the preceding claims, wherein the process after the separation in step c) includes the following further steps:

d) transesterifying the ester formed in step a) with a second alcohol, wherein this second alcohol differs from the alcohol used in step a), in a second reaction zone to obtain a second product mixture comprising at least the ester with the second alcohol, the eliminated first alcohol and unreacted second alcohol, wherein the second alcohol is used in an equimolar amount or in excess;

e) separating the ester formed with the second alcohol from the rest of the second product mixture and in particular from the eliminated first alcohol by means of one or more separation steps selected from thermal separation, extraction, crystallization or membrane separation, and recycling of the eliminated first alcohol into the first reaction zone from step a) and recycling of the unreacted second alcohol into the second reaction zone.

12. Process according to Claim 11, wherein the second alcohol used in step e) is a higher-boiling alcohol than the first alcohol.

13. Process according to Claim 11 or 12, wherein a proportion of the eliminated first alcohol is already withdrawn from the second reaction zone and recycled into the first reaction zone.

**Revendications**

1. Procédé pour la préparation d'un ester par carbonylation d'un hydrocarbure en C2-C16, le procédé comprenant les étapes suivantes :

a) transformation (carbonylation) d'un hydrocarbure en C2-C16 présentant au moins une double liaison oléfinique avec du monoxyde de carbone et avec un alcool, qui est un monoalcool ou un polyalcool (deux groupes OH ou plus) comprenant 1 à 50 atomes de carbone ou un mélange de deux monoalcools et/ou polyalcools ou plus et qui est utilisé, lorsqu'il s'agit d'un monoalcool, dans un rapport molaire avec l'hydrocarbure en C2-C16 (alcool:hydrocarbure en C2-C16) de 2 à 20 ou qui est utilisé, lorsqu'il s'agit d'un polyalcool, dans un rapport molaire avec l'hydrocarbure utilisé (hydrocarbure:polyalcool) de 2 à 20, en présence d'un système catalytique homogène, qui comprend au moins un métal des groupes 8 à 10 du système périodique des éléments ou un composé correspondant, un ligand contenant du phosphore et un acide, dans une zone de réaction avec obtention d'un mélange de produits liquide, qui comprend au moins l'ester formé par la transformation, le système catalytique homogène, une fraction légère, une fraction lourde et des alcools non transformés ;

b) réalisation d'une séparation sur membrane pour séparer le système catalytique homogène à partir du mélange de produits liquide, suite à quoi le système catalytique homogène et en outre l'hydrocarbure non transformé et/ou l'alcool non transformé, de préférence l'alcool non transformé, sont enrichis dans le rétentat et l'ester formé dans l'étape a) est enrichi dans le perméat, un matériau membranaire apte à l'OSN (Organic Solvent Nanofiltration - nanofiltration de solvant organique), stable à l'acide, c'est-à-dire stable pendant au moins 300 heures en présence de l'acide du système catalytique, qui présente au moins une couche active en séparation, qui est constituée par du PEEK (polyétheréthercétone), étant utilisé comme matériau membranaire ;

c) traitement du perméat au moyen d'une ou de plusieurs étapes de séparation, choisies parmi une séparation thermique, une extraction, une cristallisation ou une séparation sur membrane, de préférence au moyen d'une ou de plusieurs étapes de distillation, pour la séparation de l'ester formé dans l'étape a) et pour la séparation de l'hydrocarbure en C2-C16 non transformé présentant au moins une double liaison oléfinique et/ou de l'alcool non transformé et recyclage de l'hydrocarbure en C2-C16 présentant au moins une double liaison oléfinique non transformé et/ou de l'alcool non transformé dans la zone de réaction de l'étape a).

2. Procédé selon la revendication 1, le matériau membranaire selon l'invention présentant, après une utilisation dans le procédé de séparation sur membrane décrit ici dans l'étape b) d'au moins 300 heures, les deux caractéristiques suivantes :

- la rétention membranaire R pour le ligand du système catalytique homogène est d'au moins 75% et ne se dégrade pas de plus de 30% ; ou
- la perméabilité relative $P_{rel}$ présente une valeur entre 30% et 300%.

3. Procédé selon la revendication 1 ou 2, l'alcool utilisé dans l'étape a) étant un monoalcool ou un polyalcool (deux groupes OH ou plus) comprenant 1 à 15 atomes de carbone ou un mélange de deux monoalcools et/ou polyalcools ou plus.

4. Procédé selon la revendication 3, l'alcool utilisé dans l'étape a) étant choisi dans le groupe constitué par le méthanol, l'éthanol, le 1-propanol, le 1-butanol, le 1-pentanol, le 1-hexanol, le 2-propanol, le tert-butanol, le 3-pentanol, le 2-propylheptanol, le cyclohexanol, le phénol et leurs mélanges.

5. Procédé selon l'une quelconque des revendications précédentes, l'hydrocarbure en C2-C16 utilisé dans l'étape a) présentant au moins une double liaison oléfinique étant un n-alcène ou un iso-alcène comprenant 2 à 16 atomes de carbone.

6. Procédé selon l'une quelconque des revendications précédentes, le métal des groupes 8 à 10 du système périodique des éléments ou un composé correspondant du système catalytique homogène dans l'étape a) étant le palladium ou un composé de celui-ci.

7. Procédé selon l'une quelconque des revendications précédentes, le ligand contenant du phosphore du système catalytique homogène présentant une structure bidentate.

8. Procédé selon l'une quelconque des revendications précédentes, l'acide du système catalytique dans l'étape a) étant un acide de Brönsted présentant un pKa ≤ 5 ou un acide de Lewis présentant une valeur LAU supérieure à 25.

9. Procédé selon l'une quelconque des revendications précédentes, l'acide du système catalytique dans l'étape a) étant un acide de Brönsted ou un acide de Lewis, l'acide de Brönsted étant l'acide perchlorique, l'acide sulfurique, l'acide phosphorique, l'acide méthylphosphonique ou un acide sulfonique et l'acide de Lewis étant le triflate d'aluminium, le chlorure d'aluminium, l'hydrure d'aluminium, le triméthylaluminium, le tris(pentafluorophényl)borane, le trifluorure de bore, le trichlorure de bore ou un mélange de ceux-ci.

10. Procédé selon l'une quelconque des revendications précédentes, la zone de réaction pour la transformation dans l'étape a) présentant au moins un réacteur.

11. Procédé selon l'une quelconque des revendications précédentes, le procédé présentant les autres étapes suivantes après la séparation dans l'étape c) :

d) transestérification de l'ester formé dans l'étape a) avec un deuxième alcool, ce deuxième alcool se distinguant de l'alcool utilisé dans l'étape a), dans une deuxième zone de réaction avec obtention d'un deuxième mélange de produits, qui comprend au moins l'ester avec le deuxième alcool, le premier alcool dissocié et le deuxième alcool non transformé, le deuxième alcool étant utilisé en une quantité équimolaire ou en excès ;
e) séparation de l'ester formé avec le deuxième alcool du deuxième mélange résiduel de produits et en particulier du premier alcool dissocié au moyen d'une ou de plusieurs étapes de séparation, choisies parmi une séparation thermique, une extraction, une cristallisation ou une séparation sur membrane et recyclage du premier alcool dissocié dans la zone de réaction de l'étape a) ainsi que recyclage du deuxième alcool non transformé dans la deuxième zone de réaction.

12. Procédé selon la revendication 11, le deuxième alcool utilisé dans l'étape e) étant un alcool à point d'ébullition plus élevé que celui du premier alcool.

13. Procédé selon la revendication 11 ou 12, une partie du premier alcool dissocié étant déjà prélevée de la deuxième zone de réaction et recyclée dans la première zone de réaction.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013107902 A1
- EP 3121184 A2
- WO 2015110843 A1
- EP 3121186 A2

**In der Beschreibung aufgeführte Nicht-Patentliteratur**

- **JR GAFFEN et al.** *Chem,* vol. 5 (6), 1567-1583
- **J. DA SILVA BURGAL et al.** *Journal of Membrane Science,* 2015, vol. 479, 105-116